# EUROPÄISCHE PATENTSCHRIFT

(11) **EP 2 953 653 B1**
(45) Veröffentlichungstag und Bekanntmachung des Hinweises auf die Patenterteilung: **12.04.2017**
(21) Anmeldenummer: 14705984.4
(22) Anmeldetag: 05.02.2014
(51) Int. Cl.: A61L 2/00, A61L 2/03, A61C 19/00, A61C 8/00

(54) **BEHANDLUNGSSYSTEM ZUR REINIGUNG EINES MIT BIOFILM VERUNREINIGTEN BAUTEILS, INSBESONDERE EINES IMPLANTAT-TEILS**
TREATMENT SYSTEM FOR CLEANING A COMPONENT, IN PARTICULAR AN IMPLANT PART, CONTAMINATED WITH A BIOFILM
SYSTÈME DE TRAITEMENT CONÇU POUR NETTOYER UN COMPOSANT SOUILLÉ PAR UN BIOFILM, EN PARTICULIER D'UNE PARTIE D'IMPLANT

(30) Priorität: 05.02.2013 DE 102013201884
(43) Veröffentlichungstag der Anmeldung: 16.12.2015
(73) Patentinhaber: Zyfoma GmbH, 64331 Welterstadt (DE)
(72) Erfinder: BRODBECK, Urs, CH-8703 Erlenbach (CH)
(74) Vertreter: Walkenhorst, Andreas
(86) Internationale Anmeldenummer: PCT/EP2014/052271
(87) Internationale Veröffentlichungsnummer: WO 2014/122188

(56) Entgegenhaltungen:
- WO-A1-2006/006923
- DE-A1-102010 017 886
- US-A1- 2003 146 108

## Beschreibung

Die Erfindung betrifft ein Behandlungssystem zur Reinigung eines mit Biofilm verunreinigten Bauteils, insbesondere eines Implantat-Teils.

Aus der DE 10 2010 017 886 ist eine Vorrichtung zur elektrolytischen Behandlung von Salzlösungen bekannt, die später zur bioziden Anwendung gebracht werden.

Aus der nicht vorveröffentlichten deutschen Patentanmeldung mit dem Aktenzeichen 10 2012 022 593.8, deren Offenbarung voll umfänglich mit einbezogen wird ("incorporation by reference"), ist ein Behandlungselement, insbesondere zur Verwendung mit einem Implantat-Teil, sowie ein Verfahren zum Reinigen eines Dental-Implantat-Teils bekannt. Eine derartige Reinigung eines Implantat-Teils kann wünschenswert oder erforderlich sein, um den Erhalt des inserierten Implantats in der Knochensubstanz zu gewährleisten. An der von Gewebe und Gewebeflüssigkeit umschlossenen festen Oberfläche von Implantaten kann sich nämlich ein Biofilm bilden, der von Bakterien besiedelt wird, die letztlich zu chronischen und wiederkehrenden Infektionen führen können. Dieses Erkrankungsbild wird als Periimplantitis bezeichnet. Insbesondere im dentalen Bereich ist ähnlich wie bei der Parodontitis eine Kombination aus vernachlässigter Mundhygiene, Anhaftung von Biofilm an der üblicherweise mikrorauen Oberfläche des Dentalimplantats und weiterer Faktoren ursächlich für das Vollbild der Periimplantitis, welche sich durch eine zunehmende Belastung und Zerstörung des Hart- und Weichgewebes auszeichnet. Die Bereiche, an denen sich das Hart- und/oder Weichgewebe zurückzieht, werden dabei in der Regel mit einem Biofilm überzogen.

Das in der genannten Anmeldung beschriebene Reinigungsverfahren beruht auf dem Konzept, den die Verunreinigung bildenden Biofilm bzw. die Keime ausgehend von der Implantatoberfläche abzutöten und zu entfernen, ohne dabei die Implantatoberfläche zu beschädigen. Dazu ist ein elektrolytischer Prozess vorgesehen, bei dem Ionen (Kationen und/oder Anionen) mittels elektrostatischer Kräfte durch den Biofilm hindurch befördert werden. Diese Ionen reagieren an der Implantatoberfläche chemisch oder elektrochemisch. Durch diese Reaktionen werden neue Stoffverbindungen geschaffen und/oder die Ionen selbst und/oder Teile dieser Ionen in den atomaren Zustand überführt. Darüber hinaus besteht zudem die Möglichkeit, dass die Ionen mit dem Oberflächenmaterial reagieren (z. B. Bildung einer Oxidschicht oder Materialabtrag).

Die keimtötende Wirkung dieses Prozesses basiert auf unterschiedlichen Effekten. Zum einen werden durch das Anlegen einer elektrischen Spannung Ionen aus dem Biofilm selbst (auch aus den Bakterien) zur Anode oder Kathode befördert. Dies kann zur Abtötung von Bakterien und Viren führen. Darüber hinaus können die Ionen, während sie den Biofilm passieren, biochemische Reaktionen eingehen, was ebenfalls zur Abtötung von Bakterien und/oder Viren führen kann. Eine weitere Möglichkeit der Abtötung besteht darin, dass die an der Implantatoberfläche neu gebildeten Stoffverbindungen antibakterielle und/oder antivirale und/oder antifungizide Wirkung besitzen. Dies kann natürlich auch passieren, wenn die Ionen in den atomaren Zustand übergehen.

Das in der genannten Anmeldung beschriebene Behandlungselement ist spezifisch dafür ausgelegt, dieses Reinigungsverfahren direkt am inserierten Dentalimplantat durchzuführen, also bevorzugt während sich das Pfostenteil im Knochen im Patientenmund befindet. Dazu ist das Behandlungselement dazu vorgesehen, direkt mit dem inserierten Pfostenteil verbunden zu werden und sodann eine geeignete Behandlungsflüssigkeit, die bei Beaufschlagung mit elektrischem Strom als Basis für den angestrebten elektrolytischen Prozess dienen kann, in unmittelbarer Nähe zum inserierten Pfostenteil in den betroffenen Raumbereich der benachbarten Knochensubstanz auszubringen und mit dem elektrischen Strom zu beaufschlagen. Zum Einsatz dieses Behandlungselements ist jedoch die Herstellung eines sowohl mechanischen als auch elektrischen Kontakts mit dem inserierten Pfostenteil erforderlich. Hierzu muss bei der in der genannten Anmeldung beschriebenen Bauweise des Behandlungselements zum Zweck seiner Fixierung am Pfostenteil in der Regel die Prothetik am Dentalimplantat und gegebenenfalls auch dessen Abutment vorübergehend entfernt werden.

Der vorliegenden Erfindung liegt die Aufgabe zu Grunde, ein alternatives Behandlungssystem zur Reinigung eines mit Biofilm verunreinigten Bauteils, insbesondere eines Implantat-Teils, anzugeben, das eine besonders vereinfachte Handhabung und einen besonders flexiblen Einsatz ermöglicht, insbesondere ohne dass eine Demontage der Prothetik bei einem nur vergleichsweise leicht von Biofilm befallenen Implantat erforderlich wäre.

Diese Aufgabe wird erfindungsgemäß gelöst mit einem in elektrischen Kontakt mit dem behandlungsbedürftigen Bauteil bringbaren, an einen ersten Pol einer elektrischen Versorgungseinheit angeschlossenen Leiterelement und mit einer zur Ausbringung einer Behandlungsflüssigkeit vorgesehenen Medienkanüle, deren Innenraum elektrisch leitend mit dem zweiten Pol der elektrischen Versorgungseinheit verbunden ist.

Vorteilhafte Ausgestaltungen der Erfindung sind Gegenstand der Unteransprüche. Weitere und/oder alternative vorteilhafte Ausgestaltungen der Erfindung ergeben sich auch aus der Figurenbeschreibung.

Die Erfindung geht dabei von der Überlegung aus, dass eine elektrolytische Behandlung und Reinigung eines Bauteils von Biofilm auf besonders einfache und flexible Weise ermöglicht ist, indem die Bereitstellung der als Elektrolyt vorgesehenen Behandlungsflüssigkeit und die Beaufschlagung mit Strom gezielt mit Komponenten erfolgen, die eine punktgenaue und lokalisierte Anwendung ermöglichen. Eine besonders einfache Möglichkeit hierfür besteht in einer Kanüle, welche innen elektrisch leitfähig ausgeführt, aber äußerlich elektrisch isoliert ist. In der Kanüle kann dabei der Elektrolyt geführt und auch elektrisch kontaktiert werden, ohne dass ein elektrischer Kurzschluss mit der Umgebung zu befürchten wäre. An der Austrittsöffnung der Kanüle kann der Elektrolyt dann gezielt und räumlich fokussiert ausgebracht werden. Auf diesem Wege kann somit der ionische Strompfad für den elektrolytischen Reinigungsprozess gebildet werden. Die zweite Elektrode, die zur gezielten Führung des Stromflusses von dem zu behandelnden Bauteil oder Implantat-Teil selbst gebildet werden sollte, muss nun ihrerseits noch elektrisch kontaktiert werden. Dies kann über einen vorzugsweise in der Art einer Sonde ausgeführten, mit einer elektrischen Isolierung versehenen Kontaktierdraht erfolgen, der lediglich an seiner Spitze unisoliert und damit elektrisch leitfähig ausgebildet ist. Diese elektrisch leitfähige Spitze kann nun direkt auf die zu behandelnde Implantatoberfläche oder auf ein mit dem Implantat elektrisch verbundenes Prothesenbauteil (z. B. die Verbindungsschraube oder das Abutment, sofern elektrisch leitfähig) gebracht werden. Dies stellt dann den Elektronenstrompfad dar.

Über z. B. einen Schalter kann zuerst der Reinigungselektrolyt auf die Implantatoberfläche gefördert werden, um anschließend mit der Bestromung und somit der elektrolytischen Abtötung und Reinigung zu beginnen.

Um diesen Prozess und die Handhabung zu vereinfachen, können der ionische Strompfad und der Elektronenstrompfad in einem Bauteil ausgeführt sein. Hierbei können diese nebeneinander oder koaxial ineinander angeordnet sein. Die koaxiale Ausführung kann so gestaltet sein, dass der Elektronenstrompfad innerhalb des Ionenstrompfades (oder umgekehrt) ausgeführt sein kann.

In einer besonders günstigen Ausführung ist ein zur Aktivierung der Bestromung vorgesehener Schalter in unmittelbarer Nähe des Elektronenstrompfadaustritts angebracht. Hierbei besteht die Möglichkeit, die Anpresskraft des lonenstrompfadaustritts mit dem Schaltvorgang des Starts des elektrischen Abtötungs- und Reinigungsvorgangs zu koppeln. Das Ende der elektrolytischen Reinigungskanüle/des lonenstrompfadaustritts kann gerade oder gebogen sein. Die Austrittsrichtung des Elektrolyten kann in der gebotenen Variante in einem Winkel von 0° bis 180° zur Kanülenachse erfolgen. Um zu vermeiden, dass der Elektrolyt mit einem zu hohen Druck in das umliegende Gewebe gepumpt wird, besteht die Möglichkeit, am lonenstrompfadaustritt einen Reflektor und/ oder Diffusor für die Flüssigkeit anzubringen. Wird die elektrolytische Reinigungskanüle zwischen Implantat und Weichgewebe eingebracht, so könnte durch das Einströmen des Elektrolyts in dieser Tasche ein Überdruck entstehen. Zum Ausgleich dieses Überdruckes kann die elektrolytische Reinigungskanüle äußerlich mit Abflussrillen versehen sein. Die elektrolytische Reinigungskanüle kann auch zur Reinigung von Hüft-, Knie-, Schulter-, Ellbogen-, Fuß-, Zeh-, Hand-, Finger-, Wirbelsäulen- und/oder jeglicher weiterer elektrisch leitfähiger Knochenimplantate eingesetzt werden. Das Material des elektrischen Strompfadaustritts ist vorzugsweise aus einer Metalllegierung oder demselben Metall des zu reinigenden Knochenimplantats ausgeführt. Dies können sein: Titan, Zirkonium, Tantal und/oder Legierungen dieser oder anderer Metalle, welche für Knochenimplantate verwendet werden. Die elektrische Isolation beider Elektroden besteht vorzugsweise aus einem biokompatiblen Kunststoff, einem Glas oder einer Keramik.

Die mit der Erfindung erzielten Vorteile bestehen insbesondere darin, dass durch die Kombination des vorzugsweise elektrodenartigen Leiterelements einerseits mit der auf Grund der ionischen Leitfähigkeit der Behandlungsflüssigkeit einen zweiten Strompfad bildenden Medienkanüle andererseits bei hoher Flexibilität und mit hoher lokaler Präzision eine zuverlässige Reinigung auch lokal begrenzter Raumbereiche des jeweiligen Objekts vom Biofilm vorgenommen werden kann. Dabei kann zuverlässig das als besonders wirksam erkannte Reinigungskonzept der elektrolytischen Keimabtötung genutzt werden. Gerade bei nur begrenztem bakteriellen Befall des behandlungsbedürftigen Objekts kann dies besonders bedarfsgerecht erfolgen, und bei der Behandlung eines inserierten Dentalimplantats kann diese sogar vorgenommen werden, ohne dass eine Entfernung der Prothetik oder gegebenenfalls des Abutments notwendig wäre.

Ein Ausführungsbeispiel der Erfindung wird anhand einer Zeichnung näher erläutert. Darin zeigen:
- FIG. 1: ein Behandlungssystem zur Reinigung eines mit Biofilm verunreinigten Bauteils,
- FIG. 2: das Behandlungssystem gemäß FIG. 1 im vergrößerten Ausschnitt,
- FIG. 3: eine alternative Ausgestaltung des Behandlungssystems gemäß FIG. 1 im vergrößerten Ausschnitt, und
- FIG. 4: den Auslassbereich einer Medienkanüle im vergrößerten Ausschnitt.

Gleiche Teile sind in allen Figuren mit denselben Bezugszeichen versehen.

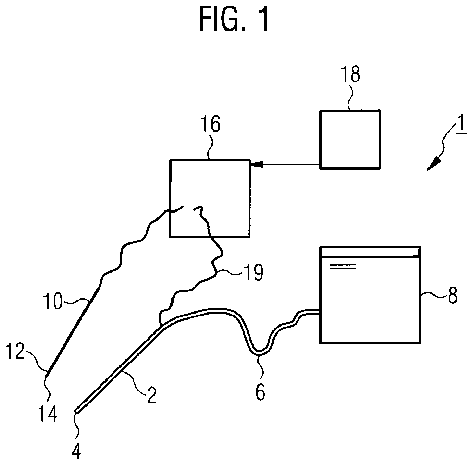

Das Behandlungssystem 1 gemäß in FIG. 1 ist zur Reinigung eines mit Biofilm verunreinigten Bauteils, insbesondere eines Implantat-Teils, vorgesehen. Das Behandlungssystem 1 ist dabei für ein elektrolytisches Reinigungskonzept ausgelegt, bei dem das behandlungsbedürftige Bauteil gezielt und lokalisiert mit einer spezifischen, geeignet gewählten Behandlungsflüssigkeit beaufschlagt und dann ein Stromfluss durch das behandlungsbedürftige Bauteil und die Behandlungsflüssigkeit erzeugt wird. Dazu umfasst das Behandlungssystem 1 eine Medienkanüle 2, in der die Behandlungsflüssigkeit geführt und über eine Auslassöffnung 4 ausgebracht werden kann. Die Medienkanüle 2 weist dabei in ihrem Endbereich eine länglich erstreckte Bauform auf, so dass eine gezielt lokalisierte und kontrollierte Ausbringung der Behandlungsflüssigkeit möglich ist. Medienseitig ist die Medienkanüle 2 über einen Verbindungsschlauch 6 mit einem Vorratsbehälter 8 für die Behandlungsflüssigkeit verbunden.

Zusätzlich ist das Behandlungssystem 1 noch als elektrisches System spezifisch ausgestaltet. Dabei ist als Auslegungsgrundsatz insbesondere vorgesehen, eine gepulste Beaufschlagung des in der Medienkanüle 2 geführten Mediums, insbesondere der darin geführten Behandlungsflüssigkeit, mit Strompulsen zu ermöglichen. Das Behandlungssystem 1 ist dabei dafür ausgelegt, den zu Reinigungszwecken des behandlungsbedürftigen Bauteils vorgesehenen Stromfluss gezielt lokalisiert im behandlungsbedürftigen Raumbereich aufbringen zu können. Das Behandlungssystem 1 ist dabei nach dem Auslegungsprinzip aufgebaut, dass der elektrische Strom dem behandlungsbedürftigen Bauteil zugeführt und dieses als Elektrode verwendet werden kann. Dazu umfasst das Behandlungssystem 1 ein einen elektrischen Strompfad bildendes Leiterelement 10. Dieses ist im Ausführungsbeispiel in der Art einer "konventionellen" Elektrode, also insbesondere als elektrisch leitfähiges nadelartiges Element aus Metall, ausgeführt, kann aber auch aus einem beliebigen anderen leitfähigen Material bestehen. Das Leiterelement 10 ist dabei an seinen Außenseiten mit einer elektrischen Isolierung versehen und weist lediglich an seinem freien Ende 12 eine frei liegende metallische Kontaktspitze 14 auf. Diese kann im Betriebsfall geeignet an das behandlungsbedürftige Bauteil angedrückt und somit ein elektrischer Kontakt zu diesem hergestellt werden. Elektrisch ist das Leiterelement 10 mit einem der Pole einer elektrischen Versorgungseinheit 16, insbesondere einer Strom- oder Spannungsquelle, verbunden.

Der elektrischen Versorgungseinheit 16 ist eine Steuereinheit 18 zugeordnet, über die der gelieferte Strom bzw. die gelieferte Spannung steuer- und einstellbar sind. Die Steuereinheit wirkt dabei zusätzlich auch auf ein nicht näher dargestelltes Fördersystem des Verbindungsschlauchs 6, mit dem die Durchflussrate der Behandlungsflüssigkeit durch den Verbindungsschlauch 6 einstellbar ist.

Zur Bildung eines Gegenpols oder der Gegenelektrode ist die Nutzung der elektrischen Leitfähigkeit der in den Medienkanüle 2 geführten Behandlungsflüssigkeit vorgesehen. Dazu ist der Innenraum der Medienkanüle 2 seinerseits über ein Kabel 19, das leitend mit dem Innenraum der Medienkanüle 2 verbunden ist, elektrisch mit dem anderen Pol der elektrischen Versorgungseinheit 16 verbunden. Damit bildet die Auslassöffnung 4 der Medienkanüle 2 in elektrischer Hinsicht einen Kontakt oder elektrischen Kontaktpunkt, über den der Stromfluss in das behandlungsbedürftige Bauteil erfolgt. Bei geeigneter Positionierung der Medienkanüle 2 und ihrer Auslassöffnung 4 möglichst in unmittelbarer Nähe zum behandlungsbedürftigen Bauteil und die Nutzung der Auslassöffnung 4 als elektrischen Kontakt ist erreicht, dass der zu Behandlungs- und Reinigungszwecken angelegte elektrische Strom durch die von den Bakterien befallene Oberflächenzone des behandlungsbedürftigen Bauteils hindurch- und von dort aus weitgehend unmittelbar, also insbesondere ohne "Umwege" über weiteres Körpergewebe oder dergleichen, zur als Kontaktfläche dienenden Auslassöffnung 4 fließen kann. Die Medienkanüle 2 einschließlich der darin geführten, elektrisch leitfähigen Behandlungsflüssigkeit und den entsprechenden Anschlusselementen bildet somit im Ausführungsbeispiel ein zweites, einen elektrischen Strompfad zur Auslassöffnung 4 bildendes Leiterelement.

Die Medienkanüle 2 ist aus einem geeignet gewählten elektrisch isolierenden Grundstoff, beispielsweise aus einem Kunststoff, gefertigt. Um dabei aber die Nutzung der in der Medienkanüle 2 befindlichen Behandlungsflüssigkeit als elektrisches Leiterelement noch weiter zu begünstigen und insbesondere eine zuverlässige elektrische Kontaktierung sicherzustellen, ist die Medienkanüle 2 innenseitig, also an ihrer der Behandlungsflüssigkeit zugewandten Innenoberfläche, mit einer elektrisch leitfähigen Beschichtung versehen.

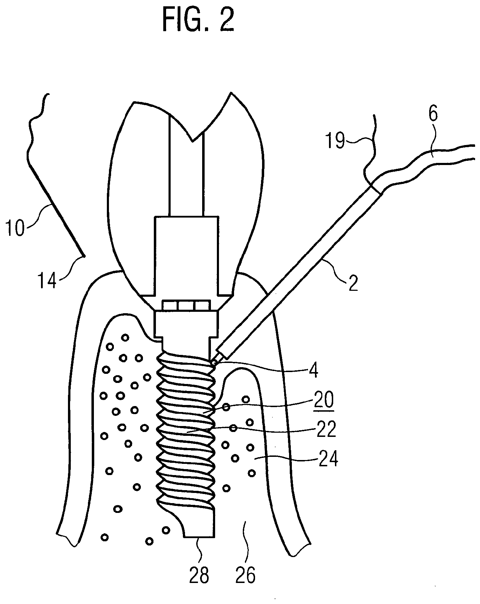

Wie der vergrößerten Darstellung gemäß FIG. 2 entnehmbar ist, ist die Medienkanüle 2 zur gezielten und lokalisierten Zuführung der Behandlungsflüssigkeit an das behandlungsbedürftige Bauteil ausgelegt. Im Ausführungsbeispiel wird dies anhand eines in den Mundknochen eines Patienten inserierten Dentalimplantats 20 erläutert; selbstverständlich sind aber auch andere Anwendungen denkbar, bei denen auf flexible und fokussierte Weise ein Bauteil, beispielsweise ein Knochenimplantat beliebiger Bauweise, vom Befall eines Biofilms gereinigt werden soll. In FIG. 2 ebenfalls dargestellt ist ein dem Dentalimplantat 20 im Bereich seines Außengewindes 22 benachbarter, räumlich begrenzter Raumbereich 24 im Kieferknochen 26, der von Periimplantitis befallen und dementsprechend mit Bakterien belastet ist.

Generell besteht bei Dental-Implantatsystemen, insbesondere auch bei zweiteiligen Implantatsystemen, das Problem, dass durch ein Eindringen von Bakterien oder Keimen in den Gewebebereich in der Nähe der Insertionsstelle, insbesondere im Bereich des in den Kiefer eingebrachten Außengewindes 22, Entzündungen oder Entzündungsherde entstehen können. Derartige, insbesondere auch in Folge einer so genannten Periimplantitis entstehende Entzündungen können, insbesondere wenn sie sich über einen längeren Zeitraum entwickeln und verfestigen können, zu einer gravierenden Beeinträchtigung des Gewebes und des Knochens im Bereich der Insertionsstelle führen. Ohne geeignete Gegenmaßnahmen können diese Beeinträchtigungen dazu führen, dass das gesamte Implantatsystem wieder aus dem Knochen entfernt und durch andere Prothetik ersetzt werden muss. Dieser durch die Periimplantitis hervorgerufene, äußerst unerwünschte Effekt kann somit zu einem Totalverlust des Implantatsystems führen, so dass erneute chirurgische Maßnahmen wie beispielsweise ein Ausschaben des betroffenen Bereichs im Kieferknochen und die Neuversorgung mit einem Implantatsystem notwendig werden können. Durch eine derartige Entnahme kann es zudem zu Knochenverlust oder sonstigem Verlust an Gewebesubstanz kommen, die im Extremfall soweit führen kann, dass eine Neuversorgung mit einem anderen Implantat gar nicht mehr möglich ist. Eine derartige durch Periimplantitis hervorgerufene Notwendigkeit einer Neuversorgung kann auch nach vergleichsweise langen Zeiträumen nach dem ersten Einsetzen des Implantatsystems von beispielsweise bis zu einigen Jahren oder sogar Jahrzehnten auftreten.

Die im Zusammenhang mit einer Periimplantitis beobachteten Keime oder Bakterien können dabei grundsätzlich das Innere der Komponenten des Dentalimplantats 20 besiedeln, haften aber in der Regel bevorzugt direkt an der Oberfläche des in den Kieferknochen 26 inserierten Dentalimplantats 20 im Kontaktbereich mit dem umgebenden Gewebe oder Knochenmaterial, also insbesondere im Bereich des Außengewindes 22, an. In dessen Bereich kann die Oberfläche des Dentalimplantats 20 mit einer Aufrauhung oder dergleichen versehen sein, um das Einwachsen in das Gewebe oder den Knochen besonders zu begünstigen und das Einheilen des Dentalimplantats 20 nach der Insertion zu unterstützen. Gerade im Bereich einer derartigen, eigentlich als besonders günstig für das Implantatsystem angesehenen Aufrauhung der Oberfläche kann aber die Ansiedlung der Keime oder Bakterien vermehrt stattfinden, wobei die Rauigkeit ein gezieltes Entfernen der vorhandenen Keime oder Bakterien noch zusätzlich erschwert.

Es besteht daher der dringende Wunsch nach geeigneten Gegenmaßnahmen, um für den Fall einer sich anbahnenden oder bereits aufgetretenen Periimplantitis unter Erhaltung des bereits eingesetzten Implantatsystems wirksam den Entzündungsherd bekämpfen und die eingedrungenen Keime abtöten zu können, so dass sich anschließend wieder gesundes Gewebe oder gesunde Knochensubstanz im Bereich um das Außengewinde 22 herum bilden kann. Dazu ist wünschenswert, zusätzlich zu einem gezielten Abtöten der Keime oder Bakterien im betroffenen Bereich auch noch deren Materialreste und Fragmente zuverlässig aus dem betroffenen Raumbereich zu entfernen, so dass anschließend der betroffene Bereich wieder von gesundem Gewebe oder Knochenmaterial ausgefüllt werden und sich wieder eine innige Verbindung zwischen der Außenoberfläche des Dentalimplantats 20 und dem umgebenden Gewebe oder Knochenmaterial bilden kann. Zudem sollte der von der Bakterienbeschichtung gebildete Biofilm einschließlich der organischen Reste abgetöteter Bakterien zuverlässig entfernt werden.

Zu diesem Zweck, also zum Abtöten von Keimen oder Bakterien im Insertionsbereich des Dentalimplantats 20 und insbesondere auch zum anschließenden Ausspülen, Entfernen und Austragen der Gewebe- und Materialreste der abgetöteten Bakterien, ist das Behandlungssystem 1 vorgesehen. Dieses beruht hinsichtlich seiner Ausgestaltung und prinzipiellen Ausführung auf zwei jeweils als eigenständig erfinderisch angesehenen Grundkonzepten: Einerseits ist es dafür ausgestaltet, die im Insertionsbereich des Dentalimplantats 20 vorhandenen Keime oder Bakterien durch gezielte Zuführung eines bakterioziden, aber für den menschlichen Organismus verträglichen Reinigungs- oder Desinfektionsmittels gezielt abzutöten. Andererseits ist es dafür ausgelegt, eventuell an der Oberfläche des Dentalimplantats 20, insbesondere im Bereich Außengewindes 22, noch anhaftende Reste oder Fragmente von Keimen und/oder Bakterien durch eine geeignete Beaufschlagung mit Strom oder Stromstößen von der Außenoberfläche des Dentalimplantats 20 abzulösen, so dass sie anschließend ausgewaschen werden können.

In einem ersten, sowohl bezüglich der Ausgestaltung des Systems als auch bezüglich der vorgesehenen Verfahrensschritte des Behandlungsverfahrens eigenständig als erfinderisch angesehenen Aspekt ist das Behandlungssystem 1 daher sowohl strukturell als auch funktional/konzeptionell dafür ausgelegt, die Behandlungsflüssigkeit zum Abtöten der Keime oder Bakterien und/oder zum Reinigen des inserierten Implantat-Teils gezielt in den Insertionsbereich des Dentalimplantats 20, insbesondere den Bereich von dessen Außengewinde 22, einzuspeisen.

In einem zweiten, ebenfalls sowohl bezüglich der Ausgestaltung des Systems und der Auswahl und Zusammensetzung der Grundbestandteile der verwendeten Behandlungsflüssigkeit als auch bezüglich der vorgesehenen Verfahrensschritte des Behandlungsverfahrens eigenständig erfinderischen Aspekt ist das Behandlungssystem 1 dafür ausgelegt, die abgetöteten Bakterien oder Keime bzw. deren Reste oder Fragmente zuverlässig von der Außenoberfläche des Dentalimplantats 20 abzulösen, so dass sie anschließend ausgespült werden können und sich nachfolgend gesundes Gewebe oder Knochenmaterial an die Oberfläche des Dentalimplantats 20 anlegen und dieses wieder vollständig in gesundes Gewebe oder Knochenmaterial einwachsen kann. Für die Ablösung der Bakterien oder Keime bzw. deren Resten oder Fragmenten von der Oberfläche ist deren Benetzung mit einer leitfähigen Behandlungsflüssigkeit unter Beaufschlagung mit gepulsten Stromstößen vorgesehen. Wie sich nämlich ebenfalls völlig überraschend herausgestellt hat, scheint gerade diese gepulste Beaufschlagung mit Stromstößen in Kombination mit geeignet gewählten Ionenkonzentrationen in der Behandlungsflüssigkeit besonders zuverlässig das Ablösen der Bakterien oder Keime bzw. von deren Fragmenten oder Resten von der darunterliegenden Oberfläche zu bewirken, selbst wenn diese aufgeraut ist und eigentlich das Anhaften organischen Materials auf Grund ihrer Oberflächenstruktur besonders begünstigt.

Dabei liegt die überraschende Erkenntnis zugrunde, dass die Beaufschlagung des Dentalimplantats 20 und insbesondere seines Pfostenteils 28 mit Strom unter Verwendung einer geeignet gewählten Behandlungsflüssigkeit im Bereich der Außenoberfläche des Pfostenteils 28, also insbesondere im Bereich des Außengewindes 22, zu einer elektrolytischen Reaktion in der Behandlungsflüssigkeit und damit gegebenenfalls zur Erzeugung von Gasblasen in unmittelbarer Nähe zur Oberfläche führt. Durch diese Gasblasenbildung an der Oberfläche des Pfostenteils 28 werden die oberflächlich anhaftenden Bestandteile oder Fragmente der Keime oder Bakterien mit abgelöst und restlos entfernt, so dass sie keine Basis und keinen Nährboden für eine Neuansiedlung von Keimen in diesen Bereichen bilden können. Zurück bleibt eine von Keimen, Bakterien oder deren Bestandteilen oder Resten befreite, angeraute und poröse Oberfläche des Pfostenteils 28, die gut als Basis für eine zukünftige Integration in das nachwachsende Knochengewebe dienen kann. Die verbleibende Oberfläche kann dabei auch durch eine Titan-Oxidschicht gebildet sein, die auch bei einem Anodisieren der Oberfläche entstehen würde.

Eine besondere Begünstigung dieser im Sinne einer zuverlässigen Reinigung der Oberfläche erwünschten Ablösung der an der Oberflächen haftenden Biofilmbestandteile aus dem inserierten Pfostenteil 28 ist durch eine vorteilhafte besonders geeignete Verfahrensführung bei der Beaufschlagung mit Strom erreichbar. Diese kann derart vorgenommen werden, dass die infolge des Stromflusses im Bereich der inserierten Oberfläche stattfindende elektrolytische Gasblasenbildung besonders verstärkt wird. Hierbei kann das Pfostenteil 28 anodisch oder auch kathodisch geschaltet werden. Insbesondere bei einer zumindest vorübergehend kathodischen Beschaltung des Pfostenteils 28 entsteht elektrolytisch induziert Wasserstoffgas, das besonders wirksam zur Gasblasenbildung beiträgt. Bei anodischer Schaltung des Pfostenteils entstehen hingegen, abhängig von der Zusammensetzung der Behandlungsflüssigkeit, Chlorgas, Sauerstoff, Stickstoff; Kohlenmonoxid und/oder Kohlendioxid. Die dadurch gebildeten Gasblasen steigen in der umgebenden Flüssigkeit auf und erzeugen dadurch Mitnahmeeffekte, über die die genannten Oberflächenbestandteile mit abgetragen und nach außen hin abgefördert werden. Beispielsweise wurde völlig überraschend beobachtet, dass bei Verwendung einer positive Ionen enthaltenden Lösung, beispielsweise einer wässrigen Salzlösung, diese Ionen sich bei einer kathodischen Beschaltung des Pfostenteils 28 an diesem abscheiden und damit die Gasblasenbildung deutlich verstärken. Beispielsweise führt die Anwesenheit von Na⁺-Ionen bei kathodischer Verschaltung des Pfostenteils 28 zu erheblicher Gasblasenbildung, da Na+ an der Kathode mit dem umgebenden Wasser zu NaOH reagiert und dabei Wasserstoff freisetzt.

In einem dritten, ebenfalls sowohl bezüglich der Ausgestaltung des Systems als auch bezüglich der vorgesehenen Verfahrensschritte des Behandlungsverfahrens eigenständig erfinderischen Aspekt ist das Behandlungssystem 1 für eine besonders einfach und effizient gehaltene Kombination der genannten Aspekte ausgestaltet. Dabei liegt das Konzept zugrunde, dass sowohl die vorgesehene Zuführung der Reinigungsflüssigkeit als auch das gezielte Ablösen der Bakterien- und Keimreste und -fragmente durch Aufbringung der genannten Strompulse in einem gemeinsamen System und somit mit besonders einfach gehaltenen Mitteln erreicht werden kann.

Die verwendete Behandlungsflüssigkeit ist im Hinblick auf diese Aspekte geeignet gewählt und zusammengesetzt. Die Auswahl und Zusammensetzung der Grundbestandteile der Behandlungsflüssigkeit ist dabei insbesondere im Hinblick auf die beabsichtigte Wirkungsweise, d. h. der Aufbringung eines elektrischen Stroms im Raumbereich der behandlungsbedürftigen Oberfläche, vorgenommen, wobei insbesondere sichergestellt ist, dass eine für diesen Zweck ausreichend hohe elektrische Leitfähigkeit in der Behandlungsflüssigkeit vorliegt. Diese soll insbesondere durch eine ausreichend hoch gewählte Ionendichte in der Behandlungsflüssigkeit sichergestellt werden. Dazu ist als ein Grundbestandteil der Behandlungsflüssigkeit ein Metallsalz vorgesehen, bevorzugt in wässriger Lösung. Dieses liefert die Ionen für den Stromtransport, und zudem können die nach der jeweiligen Elektrodenreaktion entstehenden Umsetzungsprodukte auch noch geeignete biochemische Wirkungen aufweisen. Durch die gezielte Wahl einer ausreichend hohen elektrischen Leitfähigkeit soll bei einer Durchführung des Reinigungsverfahrens an einem inserierten Implantat sichergestellt werden, dass der Stromfluss durch die Behandlungsflüssigkeit und damit durch die behandlungsbedürftigen Teile und Komponenten, nicht aber durch das Körpergewebe des Patienten erfolgt, so dass eine Gefährdung des Patienten durch einen ungewollten Stromfluss durch Weichgewebe, Knochen, Blut und/oder andere Körpermaterialien minimiert werden kann. Die elektrische Leitfähigkeit der Behandlungsflüssigkeit sollte dabei möglichst ein Vielfaches der elektrischen Leitfähigkeit von Blut, Knochen, Weichgewebe, Fettgewebe oder anderen Körpermaterialien aufweisen.

Demzufolge werden bei der Auswahl und Zusammensetzung der Grundbestandteile für die Behandlungsflüssigkeit insbesondere folgende Leitfähigkeitswerte berücksichtigt (die elektrische Leitfähigkeit σ wird dabei in der üblichen Einheit mS/cm angegeben):

| | |
|---|---|
| Haut: | 0,03 - 0.1 mS/cm |
| Knochen: | 0,06 - 0,2 mS/cm |
| Fettgewebe: | 0,20 - 1,0 mS/cm |
| Muskelgewebe: | 0,80 - 2,5 mS/cm |
| Blut: | ca. 6,7 mS/cm |
| andere Körperflüssigkeiten: | ca. 15 mS/cm |

Um das Gefährdungspotential für den Patienten geeignet gering zu halten und den Stromfluss auf die erwünschten Regionen zu begrenzen, sollte die elektrische Leitfähigkeit daher mindestens das Doppelte, bevorzugt das Fünffache, besonders bevorzugt das Zehnfache der Leitfähigkeit sonstiger Körperflüssigkeiten betragen. Daher sollte die elektrische Leitfähigkeit der Behandlungsflüssigkeit einen Wert von mindestens 30 mS/cm, vorzugsweise mindestens 75 mS/cm und besonders bevorzugt mindestens 150 mS/cm aufweisen. Im Vergleich zu Blut bedeutet dies, dass die elektrische Leitfähigkeit der Behandlungsflüssigkeit vorzugsweise mindestens etwa das Fünffache, bevorzugt mindestens etwas das Zehnfache und besonders bevorzugt mindestens etwa das Zwanzigfache der Leitfähigkeit von Blut beträgt. Messungen haben ergeben, dass beim Einsatz einer solchermaßen gewählten Behandlungsflüssigkeit die elektrische Spannung, der das Körpergewebe, das Blut, die Körperflüssigkeiten etc. ausgesetzt werden, geringer als 6 V, vorzugsweise geringer als 3 V, besonders bevorzugt kleiner als 1,5 V ist. Damit können Schädigungen für den Patienten aufgrund der gering gehaltenen Spannungen sicher ausgeschlossen werden. Zur Einhaltung einer solchen Leitfähigkeit ist insbesondere die Ionenkonzentration in der Behandlungsflüssigkeit und in den sie bildenden Grundbestandteilen ausreichend hoch gewählt; hierfür können Laugen, Säuren, Salze und/oder andere ionenbildende Stoffe oder Stoffverbindungen zum Einsatz kommen.

Bei der Auswahl und Zusammensetzung der Grundbestandteile der Behandlungsflüssigkeit ist in besonderem Maße berücksichtigt, dass die reinigende oder biofilmablösende Wirkung der elektrolytischen Behandlung einer kontaminierten Implantatoberfläche auf einer Kombination mehrerer Ursachen beruht, die möglichst ergänzend zueinander nutzbar gemacht werden sollten. Zum einen können sich beim Stromfluss durch den Elektrolyten bevorzugt im Bereich der Elektroden Gase oder Gasblasen bilden, welche eine abhebende (mechanische) Wirkung auf den Biofilm haben. Die Entstehung dieser Gase erfolgt unmittelbar an der als Elektrode dienenden Implantatoberfläche und somit zwischen dieser und dem Biofilm. Die dabei entstehenden Gasblasen beeinflussen mit ihrer Wachstumsgeschwindigkeit und ihrer maximalen Größe den Ablösungsprozess.

Als zweite Ursache für die das Implantat reinigende oder den Biofilm ablösende Wirkung des elektrolytischen Prozesses ist die zersetzende, zerstörende und auflösende Wirkung der elektrolytisch entstehenden Stoffe oder Stoffverbindungen auf die eigentliche Anhaftung des Biofilms an der Implantatoberfläche, also auf den Klebe- oder Verankerungsmechanismus, zu nennen.

Die dritte Ursache für die reinigende oder ablösende Wirkung des elektrolytischen Prozesses basiert auf materialabtragenden Effekten des Implantatmaterials, wobei Bestandteile oder Partikel des eigentlichen Implantats in dessen Oberflächenbereich aus diesem herausgelöst werden.

Die vierte Ursache für die reinigende oder ablösende Wirkung des elektrolytischen Prozesses basiert auf der Oxidschichtbildung metallischer Implantate, welche dies erlauben. Hierbei durchdringen Metallatome des metallischen Grundmaterials die evtl. schon vorhandene Oxidschicht basierend auf der angelegten elektrischen Spannung und reagieren mit Stoffen des Elektrolyts (meist Sauerstoff => Metalloxidbildung). Bei Metallen, welche keine Oxidschicht bzw. keine mechanisch stabile Oxidschicht bilden, können auch nicht-oxydische Stoffverbindungen entstehen (meist Salze), die dann in Lösung gehen.

Die für die Bildung der Behandlungsflüssigkeit vorgesehenen Grundbestandteile sind im Hinblick auf diese Effekte geeignet gewählt und miteinander kombiniert. Zudem ist als grundlegendes Auslegungsziel berücksichtigt, dass keine toxischen oder anderweitig einen Patienten gefährdenden oder für ihn unangenehmen Effekte auftreten sollten, so dass die Behandlungsflüssigkeit auch für einen Einsatz am inserierten Dentalimplantat, also im Patientenmund, geeignet ist. Im Ausführungsbeispiel sind dabei als Grundbestandteile mindestens ein Salz einerseits und eine Säure andererseits, bevorzugt verdünnt mit Wasser, vorgesehen, deren Auswahl und Zusammensetzung sich insbesondere nach den genannten Kriterien richtet. Besonders bevorzugt ist dabei als Säure Phosphorsäure, Zitronensäure, Ameisensäure, Essigsäure, Milchsäure, Kohlensäure oder eine Kombination von diesen vorgesehen. Alternativ oder zusätzlich besonders bevorzugt ist dabei als Salz Natrium-, Calcium-, Aluminium-, Magnesium-, Zinn- oder Kaliumiodid, -chlorid, -nitrat, -carbonat oder -hydrogencarbonat und/oder Ammoniumchlorit, -nitrat oder -iodid oder eine Kombination von diesen vorgesehen.

Des Weiteren ist dabei berücksichtigt, dass der vorgesehene elektrolytische Prozess wahlweise mit anodischer oder mit kathodischer Schaltung des Dentalimplantats geführt werden kann. Demzufolge wird im Folgenden in eine Anodenreaktion und eine Kathodenreaktion unterteilt.

Bei einer Anodenreaktion, also bei anodischer Schaltung des Dentalimplantats 2, werden an der Anode die in der Behandlungsflüssigkeit vorhandenen Anionen generell durch den Entzug von Elektronen oxidiert. Dabei kann es zu einer unmittelbaren Reaktion mit dem Material kommen, insbesondere zur Bildung einer Oxidschicht und/oder eines Salzes mit dem Implantatmaterial. Knochenimplantate und dementsprechend auch das Dentalimplantat 2 bestehen meist aus Titan, Zirkonium, Tantal oder aus Legierungen dieser Metalle. Darüber hinaus werden noch andere Metalle hinzulegiert. Diese Metalle oder Metalllegierungen weisen meist einen hohen Grad an Oxidschichtbildungen auf. Diese Oxidschichtbildung hat eine passivierende Wirkung auf die Oberfläche. Die Folge darauf ist die Unterbindung oder zumindest sehr starke Reduzierung der Anodenreaktion dieser Metalle oder Metalllegierungen. Da sich im Biofilm meist Stoffverbindungen mit Sauerstoff befinden, ist eine Unterbindung dieser Passivierung meist nicht möglich. Sollte das Dentalimplantat anodisch geschaltet sein, so ist der ablösende reinigende Effekt somit meist auf die Oxidschichtbildung beschränkt. Bei höheren Betriebsspannungen von beispielsweise mehr als 20 V konnte bei umfangreichen Untersuchungen zwar aufgezeigt werden, dass ein materialabtragender Prozess möglich ist, dass dieser aber mit einer starken Wärmeentwicklung verbunden ist. Diese Wärmeentwicklung kann zur unerwünschten Nekrose des Knochens führen. Darüber hinaus verändert der damit einhergehende Materialabtrag auch in unerwünschter Weise die Eigenschaften der ursprünglichen Implantatoberfläche.

Als Ausnahme hierzu hat sich überraschend gezeigt, dass bei einem Basismaterial des Pfostenteils 28, bei dem Aluminium als Legierungsbestandteil vorhanden ist (beispielsweise bei Titan grade5, das etwa 6 % Aluminiumanteil und 4 % Vanadiumanteil aufweist) auch eine anodische Bestromung des Pfostenteils 28 möglich ist, ohne dass die Bildung einer Oxidschicht den Prozess zu sehr behindert. Hierdurch kann, je nach Zusammensetzung der Behandlungsflüssigkeit, Chlor- oder lodgas oder auch CO₂ direkt an der Oberfläche des Pfostenteils 28 erzeugt und somit unmittelbar für die angestrebte Ablösung des Biofilms nutzbar gemacht werden. Für eine derartige Verfahrensführung ist das Behandlungselement 30 besonders vorteilhaft mit einer leitfähigen Oberflächenbeschichtung versehen, beispielsweise aus DLC ("diamond like carbon"), einem Metall, leitfähigem Kunststoff.

Aus den genannten Gründen ist das Pfostenteil 28 bei der Behandlung mit der Behandlungsflüssigkeit jedoch im Allgemeinen bevorzugt kathodisch geschaltet. Dabei wandern positiv geladene Ionen (Kationen) zur Oberfläche des Dentalimplantats 20. Dies können insbesondere H⁺-Ionen, Metallionen oder langkettige Kohlenwasserstoffionen, z. B. aus ionischen Flüssigkeiten. Das als Grundbestandteil für die Behandlungsflüssigkeit vorgesehene Salz ist dabei insbesondere gezielt im Hinblick auf die Eigenschaften der Kationen ausgewählt, die den genannten Prozess begünstigen oder überhaupt ermöglichen sollen. Zur Erzeugung einer möglichst hohen elektrischen Leitfähigkeit eignen sich insbesondere kleine Ionen (H⁺-Ionen oder Metallkationen), die zudem in der Art eines weiteren besonders günstigen Effekts den gegebenenfalls vorhandenen Biofilm vergleichsweise leicht durchdringen können. H⁺-Ionen werden an der durch das Dentalimplantat 20 gebildeten Kathode zu elementarem Wasserstoff H reduziert. Dies erzeugt eine Blasenbildung.

Alkalimetalle, Erdalkalimetalle und/oder Aluminium reagieren an der Kathode mit dem umgebenden Wasser zu elementarem Wasserstoff und seinem Metallkationen und OH⁻-Ionen. Dies bedeutet, dass sich Wasserstoffblasen und das Hydroxid des verwendeten Metallions bilden. Durch die Kombination dieser Komponenten ist somit neben der ablösenden Wirkung des entstehenden Wasserstoffs erreicht, dass das Metallhydroxid antibakteriell wirkt und einen verdünnenden oder auflösenden Einfluss auf den Biofilm bzw. dessen Adhäsionsmechanismus ausübt.

Um Unverträglichkeiten mit dem Körpergewebe zu vermeiden, sind als Metallkationen insbesondere die körpereigenen (z. B. Kalium- und/oder Natriumionen) besonders bevorzugt. Darüber hinaus eignen sich auch Calcium, Magnesium und/oder Aluminiumionen. Das als Grundbestandteil für die Behandlungsflüssigkeit vorgesehene Salz ist daher besonders bevorzugt ein Salz dieser Metalle, insbesondere da diese Metallkationen ohnehin nur in Form eines Salzes, z. B. in Wasser gelöst, zur Verfügung gestellt werden können.

Diese Metallsalze können Verbindungen der genannten Metalle mit einem geeigneten Salzbildner, beispielsweise mit Schwefel, Phosphor, Stickstoff, Fluor, Chlor, Jod, Brom, Kohlenwasserstoff, Sauerstoff, Bor oder anderen Nichtmetallen, sein. Der Salzbildner wird dabei vorteilhafterweise unter Berücksichtigung des Grundsatzes "je größer das Anion, desto geringer die elektrische Leitfähigkeit" und im Hinblick auf die grundsätzlich gewünschte hohe elektrische Leitfähigkeit geeignet gewählt. Als Anion werden zudem bevorzugt nur Stoffe in Betracht gezogen, welche weder die Gesundheit noch das periimplantäre Gewebe beeinflussen. Weiterhin gilt zu beachten, dass unangenehme Gerüche oder Geschmacksverbindungen unerwünscht sind. Aus diesen Gründen werden Schwefelanionen oder Anionen, die Schwefel in Kombination mit Sauerstoff oder anderen Elementen enthalten, als eher ungeeignet angesehen. Dies gilt auch für Fluor-, Brom-, Stickstoff- und Borionen, gegebenenfalls auch in Kombination mit weiteren Elementen.

Demgegenüber haben Phosphate, Phosphationen und Hydrogenphosphationen meist keine oder kaum eine schädliche Wirkung. Chlorionen oder Ionen, welche Chlor enthalten, haben meist eine antibakterielle Wirkung. Sollte das Chlorion allerdings elektrolytisch oxidiert werden und in Wasser elementar vorhanden sein, so bildet sich dabei Salzsäure und hypochlorige Säure. Dies würde zwar in Kombination mit dem kathodisch entstandenen Hydroxid zu einer Neutralisierung führen. Jedoch hat sich bei Untersuchungen gezeigt, dass das Chlor, welches an der Gegenelektrode zum Implantat (Anode) entsteht, in großem Maße dem Elektrolyten als Gas entweicht. Sollte das Chlor bei der Behandlung nicht restlos abgesaugt werden können, kann es zu starken Verätzungen in der Lunge und/oder den Schleimhäuten kommen. Hierbei gilt es abzuwägen, ob der Nutzen für den Patienten oder dessen Gefährdung größer ist.

Zu den Phosphaten des Aluminiums, des Kaliums, des Natriums, des Calciums oder des Magnesiums ist zudem zu bemerken, dass die Löslichkeit in Wasser so gering ist, dass eine ausreichende elektrische Leitfähigkeit des Elektrolyten nicht gewährleistet ist (diese Phosphate eignen sich allerdings sehr gut als Zusatzstoffe des Elektrolyten zur Pufferung des pH-Wertes). Chloride der vier eben aufgeführten Metalle hätten zwar eine ausreichende Löslichkeit in Wasser und eine gute Reinigungs- und Abtötungswirkung auf den Biofilm, können aber nicht als das Optimum angesehen werden. Bei Nitraten und/oder Nitriten ist eine Gefährdung des Patienten durch die Bildung von NOₓ-Gasen zu erwarten. Aus diesem Grund ist vom Einsatz von Nitriten oder Nitraten abzuraten.

Im Hinblick auf die genannten Auslegungsziele, insbesondere für eine besonders gute Verträglichkeit für den Patienten, ist in bevorzugter Ausführung als Salzbildner Jod vorgesehen. Besonders vorteilhaft ist dabei, dass Jodsalze des Kaliums und des Natriums auch im menschlichen Körper von Natur aus vorhanden sind. Bei der Oxidation von Jodionen an der Anode entsteht zunächst elementares Jod, welches sich in eine Natriumiodid-/Kaliumiodidlösung lösen kann. Es entsteht dabei eine Jod-Kalium-Jodid-Lösung bzw. eine Jod-Natrium-Jodid-Lösung. Beide Lösungen sind starke Desinfektionsmittel, welche sich in der Humanmedizin bewährt haben.

Reine Natrium- oder Kaliumiodidlösungen oder ein Gemisch aus beiden haben jedoch als möglichen Nachteil die Natrium- und/oder Kaliumhydroxidbildung und den damit verbundenen pH-Wertanstieg zur Folge. Als problematisch könnte nämlich ganz allgemein bei der bereits oben beschriebenen Bildung von Metallhydroxid einzustufen sein, dass ein Metallhydroxid den pH-Wert des Elektrolyten erhöht. Ein solchermaßen erhöhter pH-Wert und die sich bildende Lauge oder Base des gelösten Metallhydroxids könnten einen unerwünschten Einfluss auf das umliegende Gewebe im Patientenmund und insbesondere den Knochen haben. Auch könnten umliegende Zähne geschädigt werden. Zudem könnte die Bildung von Hydroxiden dazu führen, dass diese sich aufgrund ihrer sehr geringen Wasserlöslichkeit auf dem Pfostenteil 28 oder allgemein auf dem behandlungsbedürftigen Bauteil niederschlagen und damit den weiteren Stromfluss und somit den Prozess insgesamt behindern. Allenfalls bei Verwendung eines Calciumsalzes in der Behandlungsflüssigkeit würde das entstehende Calciumhydroxid, das als Bestandteil des Knochenmaterials vorkommt, in den Knochen integriert werden können; Calcium ist somit besonders bevorzugter Bestandteil des Salzes. Um diese unerwünschten Einflüsse zu kompensieren, weist die Behandlungsflüssigkeit als weiteren Grundbestandteil in der Art eines pH-Puffers oder -Reduzierers die Säure auf.

Die Säure ist dabei ihrerseits gezielt in der Art eines Auslegungskriteriums derart gewählt, dass sie möglichst nicht den Patienten oder das periimplantäre Gewebe gefährdet, sondern zum einen das Hydroxid neutralisiert (und auch den pH-Wert möglichst nicht über 7 steigen lässt), wobei zum anderen die Reaktionsprodukte dem eigentlichen Ziel der Reinigung des Implantatkörpers und der Ablösung des Biofilms dienen sollten. Als Mineralsäuren kommen dazu bevorzugt Phosphorsäuren und/oder Phosphatsäuren in Frage. Diese sind in ihrer Konzentration aus gesundheitsgefährdenden und/oder knochen-/gewebegefährdenden Gründen limitiert. Eine besonders bevorzugte Säure, welche auch als Mineralsäure angesehen wird und einen besonders positiven Effekt auf das Gesamtziel der Abtötung und Reinigung hat, ist hingegen die Kohlensäure. Diese ist jedoch in ihrer anwendbaren Menge durch das vergleichsweise geringe Lösungsvermögen in Wasser limitiert.

Organische Säuren stellen demgegenüber ähnlich wie Mineralsäuren pH-Wert-reduzierende und Hydroxid neutralisierende H⁺-Ionen zur Verfügung. Da sie zudem keine oder allenfalls geringfügige Schädigungen im Gewebe erzeugen oder beim Patienten insgesamt hervorrufen, sind derartige organische Säuren als Grundbestandteil für die Behandlungsflüssigkeit ganz besonders bevorzugt. Organische Säuren wären z. B. Alkansäuren, Fruchtsäuren, Carbonsäuren sowie Hydroxylcarbonsäuren. Als besonders geeignete Säuren haben sich α-Hydroxylcarbonsäuren herausgestellt. Insbesondere zeigen die besonders bevorzugten Säuren Milchsäure, Zitronensäure und Apfelsäure keinerlei gesundheitsschädigende Effekte auf den Patienten generell oder auf das periimplantäre Gewebe. Speziell bei stark mit Biofilm behafteten und kontaminierten Implantaten, auf welchen sich auch Zahnstein gebildet hat, zeigten bereits vergleichsweise geringe Dosierungen an Essigsäure einen guten Reinigungserfolg. Weitere Säuren, welche die Reinigung sowie den bakterientötenden Effekt aufweisen, aber aus gesundheitlichen Gründen nicht unbedenklich sind, wären die Fumarsäure, Gluconsäure, Glycolsäure, Salicylsäure, Mandelsäure, Weinsäure, Essigsäure, Oxalsäure und Ameisensäure.

Bei der Neutralisierung des Hydroxidions OH- mit dem entsprechenden H⁺-Ion einer Säure entsteht zusätzlich das Metallsalz der verwendeten Säure des entsprechenden Metallhydroxids. Der vorgesehene Einsatz der Säure ist somit nicht nur zur Pufferung des pH-Werts vorteilhaft, sondern trägt überdies noch zur Umwandlung des vergleichsweise schlecht wasserlöslichen Hydroxids in vergleichsweise gute wasserlösliche Salze bei und verhindert somit die Abscheidung unerwünschten und für den Prozess hinderlichen Niederschlags auf dem behandlungsbedürftigen Bauteil. Die genannten Salze werden insbesondere bei der Kombination der genannten bevorzugten Materielaien u. a. auch in der Medizin eingesetzt. Bei der Neutralisation des Kalium-, Natrium- und/oder Calciumhydroxids mit Milchsäure bildet sich das Kaliumlaktat (es besitzt eine breitbandige antimikrobielle Wirkung), Natriumlaktat bzw. Calciumlactat. Werden die sich bildenden Hydroxide hingegen mit Zitronensäure neutralisiert, bilden sich Zitrate des Kaliums, Natriums bzw. Calciums. Gerade für Natriumzitrat ist dies besonders vorteilhaft, da dieses die Blutgerinnung verhindert. Dies ist besonders vorteilhaft, da während des Prozesses austretendes, auf der Implantatoberfläche gerinnendes Blut die Ionenwanderung zur Implantatoberfläche und damit die Fortsetzung des Behandlungsprozesses insgesamt behindern könnte.

Bei der Neutralisation der Hydroxide mit Apfelsäure entstehen hingegen Malate des jeweiligen Kations, welche ebenfalls günstige Auswirkungen auf den Prozess haben. Bei der Neutralisation der Hydroxide mit Essigsäure entstehen Acetate des Kaliums, Natriums und/oder Calciums, welche ebenfalls einen günstigen Einfluss auf den Prozess haben.

Die Lactate, Zitrate, Malate und/oder Acetate des Kaliums, Natriums und/oder Calciums besitzen alle eine säureregulierende Wirkung und sind so verträglich, dass sie nach den jetzigen EU-Verordnungen für Lebensmittelzusatzstoffe in ihrer Verwendung an keine Mengenlimitierung gebunden sind.

Bei der Verwendung von Säuren in dem Elektrolyten in Kombination mit Jodiden und/oder Chloriden des Natriums, Kaliums, Magnesiums, Aluminiums und/oder Calciums hat sich bei der elektrolytischen Anwendung überraschend herausgestellt, dass durch die direkte Reduzierung des H⁺-Ionen die Blasenbildung derart positiv beeinflusst wird, dass sich der Biofilm deutlich schneller und besser ablöst. Dabei entsteht mit hoher Erzeugungsrate eine Vielzahl vergleichsweise kleiner Bläschen, die aufgrund ihrer vergleichsweise geringen Größe den Biofilm als Ganzes und nicht nur lokal von der darunterliegenden Oberfläche lösen können. Dadurch wird der Biofilm bevorzugt als Ganzes oder in vergleichsweise großen, zusammenhängenden Stücken statt in einer Vielzahl kleinerer Fragmente abgehoben, was eine deutlich verbesserte Reinigungswirkung bedingt.

Anstelle von Metallkationen können auch Ammoniumkationen verwendet werden. Hierbei besteht allerdings die Gefahr, dass bei dem elektrolytischen Prozess andere Ammoniumverbindungen (z. B. Ammoniak) gebildet werden. Dies stellt eine Gefährdung des Patienten dar und zeigt sich auch durch einen sehr unangenehmen Geschmack und Geruch.

Es wurde bei Versuchen beobachtet, dass sich der Biofilm teilweise in sehr kleinen Fragmenten oder aber in größeren zusammenhängenden Teilen ablöst. Letzteres wird bevorzugt, da damit vergleichsweise großflächig sehr günstige Reinigungsergebnisse erzielt werden können. Untersuchungen haben weiterhin gezeigt, dass der Abtransport des gelösten Biofilms und/oder seiner Fragmente durch eine Schaumbildung an der Implantatoberfläche begünstigt wird. Es hat sich herausgestellt, dass es günstig ist, dass nach dem Einsatz eines Elektrolyten aus den beschriebenen Metallsalzen, Säuren und Wasser, die insbesondere für die Abtötung und Ablösung zuständig sind, ein zweiter Elektrolyt zum Einsatz kommt, welcher zusätzlich eine Schaumbildung im Bereich der Kathode aufweist. Eine solche Schaumbildung kann erreicht werden, indem dem Elektrolyten bevorzugt zusätzlich ein Stoff zugegeben wird, der mindestens drei CH₂-Kettenglieder oder mindestens ein CH₂-Kettenglied und mindestens eine Kohlenstoffringverbindung aufweist. Hierbei können z. B. Öl und/oder Chlorhexidin zum Einsatz kommen. Darüber hinaus können auch ionische Flüssigkeiten verwendet werden, welche vorzugsweise ein I⁻-, Cl⁻- und/oder OH⁻-Ionen aufweisen. Da der organische Kationenanteil einer ionischen Flüssigkeit unter Umständen auf der Implantatoberfläche reduziert wird und dort verbleicht, ist es in einer besonders günstigen Ausführung möglich, Knochenwachstumsfaktoren an diesen Kationenanteil anzuhängen.

Werden Chloride und Jodide im richtigen Verhältnis gemischt, kann die störende Chlorgasbildung vermieden werden. An der Anode entsteht:

2J + 5Cl + 6H₂O → 10HCl + 2HlO₃

Dies bedeutet, dass an der Anode Salzsäure sowie Jodsäure gebildet werden. Diese haben sicherlich eine starke antimikrobielle Wirkung und werden auch beim Zusammentreffen mit dem sich kathodisch bildenden Hydroxid wieder neutralisiert.

Eine ganz besonders bevorzugte Zusammensetzung der Behandlungsflüssigkeit, die im Laborversuch besonders günstige Reinigungseigenschaft zeigte, umfasst eine wässrige Lösung von Natriumiodid (Nal) oder Kaliumiodid (Kl) in einem Mischungsverhältnis von mindestens 5, bevorzugt mindestens 10, besonders bevorzugt mindestens 20 g des Salzes pro 30 ml Flüssigkeit (d. h. Wasser, H₂O, gegebenenfalls mit CO₂ angereichert), durch Zugabe von Milchsäure gepuffert auf einen pH-Wert von etwa 2,7 bis 2,9.

Bei der Prozessführung ist eine mittlere Stromdichte am Pfostenteil 28 bzw. am behandlungsbedürftigen Bauteil von mindestens 50 mA/cm², vorteilhafterweise von mindestens 100 mA/cm², besonders bevorzugt von mindestens 250 mA/cm², vorgesehen, wobei diese Stromdichte auf die äußere Oberfläche des Pfostenteils 28 (also ohne Berücksichtigung von oberflächenvergößernden Eigenschaften wie beispielsweise Rauigkeit oder Oberflächenstruktur) bezogen ist. Zur Ablösung des Biofilms hat sich eine durchschnittliche Stromdichte von 50 mA/cm² bis 300 mA/cm², vorteilhafterweise von 100 mA/cm² bis 200 mA/cm² als besonders günstig erwiesen. Zum Abtransport der Biofilmfragmente sollte die mittlere Stromdichte vorzugsweise auf den Bereich von 300 mA/cm² bis 5.000 mA/cm² oder besonders vorteilhaft von 1.500 mA/cm² bis 2.000 mA/cm² erhöht werden.

Eine besonders vorteilhafte Weiterentwicklung des Systems insgesamt, d. h. des elektrolytischen Reinigungskonzepts, sieht zusätzlich die Beaufschlagung der Behandlungsflüssigkeit mit Ultraschall vor. Damit können die Reinigungseffekte neben ihrer chemischen Grundlage noch auf mechanische Weise unterstützt werden, so dass die erreichbare Reinigungswirkung deutlich erhöht werden kann. Zu diesem Zweck kann der Reinigungskanüle an geeigneter Position ein Ultraschall-Generator zugeordnet sein.

In der in FIG. 2 dargestellten Ausführungsform sind die Medienkanüle 2 einerseits und das Leiterelement 10 andererseits als voneinander im Wesentlichen unabhängige Bauteile ausgeführt, die elektrisch mit der gemeinsamen Versorgungseinheit 16 verbunden sind. Das Leiterelement 10 ist dabei im in FIG. 2 gezeigten Ausführungsbeispiel in der Art einer "konventionellen" Elektrode, also insbesondere als elektrisch leitfähiges nadelartiges Element aus Metall, ausgeführt. Durch diese Ausführung können Medienkanüle 2 einerseits und Leiterelement 10 andererseits unabhängig voneinander bewegt und positioniert werden, so dass eine besonders flexible Bearbeitung des behandlungsbedürftigen Bauteils ermöglicht ist. Insbesondere können dabei die Kontaktspitze 14 einerseits und die Auslassöffnung 4 andererseits unabhängig voneinander und damit gegebenenfalls für einen optimierten elektrischen Kontakt zum Bauteil geeignet an diesem positioniert werden.

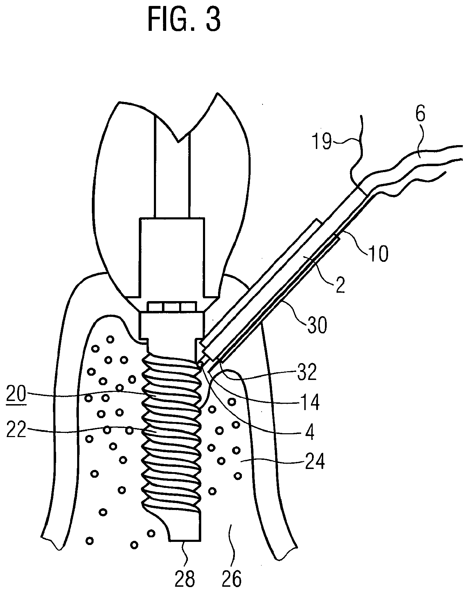

Demgegenüber ist im Ausführungsbeispiel gemäß FIG. 3 eine Variante gezeigt, bei der die Medienkanüle 2 und das Leiterelement 10 in ein gemeinsames Gehäuse 30 integriert sind. Im gezeigten Ausführungsbeispiel sind der Medienkanal in der Medienkanüle 2 einerseits und der elektrische Leiter des Leiterelements 10 andererseits benachbart zueinander und nebeneinander geführt, so dass die Kontaktspitze 14 und die Auslassöffnung 4 entsprechend benachbart zueinander am freien Ende 32 des Gehäuses 30 positioniert sind. Alternativ ist auch eine koaxiale Ausführung dieser Komponenten denkbar, bei der der Medienkanal in der Medienkanüle 2 das Leiterelement 10 (oder umgekehrt) ringförmig umgibt. Im Allgemeinen ist durch eine derartige integrierte Bauweise mit dem für Medienkanüle 2 und Leiterelement 10 gemeinsamen Gehäuse 30 eine erleichterte Handhabung erreichbar, da für den Behandler damit auch eine einhändige Bedienung und Positionierung des Systems möglich ist.

Das Behandlungssystem 1 und insbesondere dessen Steuereinheit 18 ist für eine koordinierte Verfahrensführung in dem Sinne ausgelegt, dass die Einspeisung der Behandlungsflüssigkeit einerseits und die Strombeaufschlagung andererseits abgestimmt aufeinander vorgenommen werden. Dazu kann beispielsweise vorgesehen sein, dass über die Steuereinheit 18 eine koordinierte Ansteuerung der dem Verbindungsschlauch 6 oder dem Vorratsbehälter 8 zugeordneten Fördereinheit für die Behandlungsflüssigkeit einerseits und der elektrischen Versorgungseinheit 16 andererseits erfolgt. Dies kann automatisiert oder bedarfsweise auch manuell über einen Schalter gesteuert sein. Ein manuell betätigbarer Schalter kann dabei insbesondere im Bereich des Endstücks der Reinigungskanüle angeordnet sein, so dass der Bediener beim Behandeln des Patienten Zugriff auf die Systemsteuerung nehmen kann.

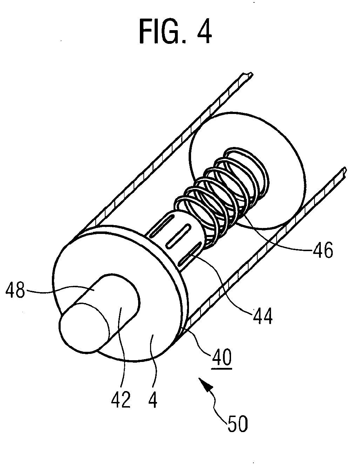

Für eine besonders erleichterte Handhabung ist die Auslassöffnung 4 der Medienkanüle 2, wie dies in der ausschnittsweisen Vergrößerung gemäß FIG. 4 dargestellt ist, mit einem Verschluss 40 versehen, der selbsttätig dann öffnet, wenn die Auslassöffnung 4 mit einer Kraft von mehr als einer vorgebbaren Mindestkraft gegen eine Fläche angedrückt wird. Dazu umfasst im Ausführungsbeispiel gemäß FIG. 4 der Verschluss 40 einen in Längsrichtung des Medienkanals in der Medienkanüle 2 verschiebbar gelagerten Hohlzylinder 42, dessen Zylindermantel mit einer Anzahl von Einströmöffnungen 44 versehen ist. Der Hohlzylinder 42 ist über eine Vorspannfeder 46 derart abgestützt, dass er im unbelasteten Zustand innerhalb einer im Bereich der Auslassöffnung 4 angeordneten Führungshülse 48 positioniert ist, die die Einströmöffnungen 44 verschließt. Wenn der Hohlzylinder 42 jedoch mit ausreichend hoher Kraft von mehr als der Federkraft an eine Fläche angedrückt wird, wird er entgegen der Federkraft nach innen in die Medienkanüle 2 hinein verschoben, so dass die Einströmöffnungen 44 frei gegeben werden und das Medium aus der Medienkanüle 2 durch den Hohlzylinder 42 hinaus ausströmen kann. Durch eine derartige oder ähnliche Bauweise ist erreicht, dass der Bediener durch Andrücken der Medienkanüle an eine Kontaktfläche die Abgabe der Behandlungsflüssigkeit direkt steuern kann.

In vorteilhafter Weiterbildung ist dieses System elektrisch mit der Steuereinheit 18 der elektrischen Versorgungseinheit 16 gekoppelt. Dabei kann insbesondere eine Systemauslegung vorgesehen sein, bei der die Verschiebung des Hohlzylinders 42 und damit die Freigabe des Auslasskanals für die Behandlungsflüssigkeit elektrisch, elektronisch oder auf ähnliche Weise erfasst und das entsprechende Signal als Auslösesignal für die Steuereinheit 18 verwendet wird. Anhand dieses Auslösesignals kann sodann die Bestromung der Komponente, beispielsweise nach einem hinterlegten Bestromungsmuster, vorgesehen sein.

Der Auslassöffnung 4 ist zudem ein Verwirbler, Reflektor oder Diffusor 50 zugeordnet, um zu vermeiden, dass der Elektrolyt oder die Behandlungsflüssigkeit mit einem zu hohen Druck in das umliegende Gewebe gepumpt wird. Wird nämlich die elektrolytische Reinigungskanüle zwischen Implantat und Weichgewebe eingebracht, so könnte durch das Einströmen des Elektrolyts in dieser Tasche ein Überdruck entstehen. Zum Ausgleich dieses Überdruckes kann die elektrolytische Medienkanüle 2 äußerlich zudem mit Abflussrillen oder dergleichen versehen sein.

Die elektrolytische Reinigungskanüle oder Medienkanüle 2 kann auch zur Reinigung von Hüft-, Knie-, Schulter-, Ellbogen-, Fuß-, Zeh-, Hand-, Finger-, Wirbelsäulen- und/oder jeglicher weiterer elektrisch leitfähiger Knochenimplantate eingesetzt werden. Das Material des elektrischen Strompfadaustritts ist vorzugsweise aus einer Metalllegierung oder demselben Metall des zu reinigenden Knochenimplantats ausgeführt. Dies können sein: Titan, Zirkonium, Tantal und/oder Legierungen dieser oder anderer Metalle, welche für Knochenimplantate verwendet werden. Die elektrische Isolation beider Elektroden besteht vorzugsweise aus einem biokompatiblen Kunststoff, einem Glas oder einer Keramik.

Die elektrische Versorgungseinheit 16 und/oder die dieser zugeordnete Steuereinheit 18 ist für die vorgesehene Verfahrensführung geeignet ausgelegt. Die von dieser gelieferte Spannung oder der Strom kann als Gleichspannung bzw. -strom, von der Polarität in beide Richtungen oder als Wechselspannung an die beiden Elektroden angelegt werden. Handelt es sich um eine Wechselspannung, kann diese als Sinus, Dreieck, Rechteck oder jede erdenkliche Überlagerung dieser mit unterschiedlichen Frequenzen sein. Darüber hinaus kann diese Wechselspannung von einer Gleichspannung überlagert sein. Es besteht auch die Möglichkeit, eine pulsierende Gleichspannung zu verwenden.

Wie vorstehend beschrieben, ist es besonders vorteilhaft, mehrere unterschiedlich zueinander zusammengesetzte Elektrolyte entweder nacheinander oder gleichzeitig an oder auf das Implantat zu bringen. Dazu ist das Behandlungssystem 1 geeignet ausgeführt. Insbesondere können mehrere Vorratsbehälter 8 für mindestens zwei Flüssigkeiten oder Elektrolyte vorgesehen sein. Diese können über Pumpen und über eine oder mehrere Ventile oder Ventileinheiten gleichzeitig (mischend) oder nacheinander über den Verbindungsschlauch 6 in die Medienkanüle 2 gefördert werden.

Das Elektrodenmaterial kann aus dem gleichen Material wie das Dentalimplantat 20 sein. Da die Dentalimplantate 20 bevorzugt aus Titan oder einer Titanlegierung hergestellt sind, ist es bevorzugt, die weitere/weiteren Elektroden aus einem anderen Metall auszuführen. Titan und dem Titan ähnliche Metalle bilden bei der anodischen Bestromung meist eine schützende Oxidschicht, welche als Isolator fungiert. Um bei kathodischer Bestromung des Dentalimplantats 20 nicht über eine solche Oxidschicht den Stromfluss einzuschränken, ist es vorteilhaft, ein nicht oder kaum eine Oxidschicht bildendes Metall als Gegenelektrode zu verwenden. In einem besonders günstigen Fall korrodiert diese Elektrode weder bei Kontakt mit den Medien/Elektrolyten noch bei der Beaufschlagung mit einer Spannung oder einem Strom. Vorzugsweise ist diese Elektrode aus Gold, Platin, Palladium ausgeführt.

### Bezugszeichenliste

- 1: Behandlungssystem
- 2: Medienkanüle
- 4: Auslassöffnung
- 6: Verbindungsschlauch
- 8: Vorratsbehälter
- 10: Leiterelement
- 12: Ende
- 14: Kontaktspitze
- 16: Versorgungseinheit
- 18: Steuereinheit
- 19: Kabel
- 20: Dentalimplantat
- 22: Außengewinde
- 24: Raumbereich
- 26: Kieferknochen
- 28: Pfostenteil
- 30: Gehäuse
- 32: Ende
- 40: Verschluss
- 42: Hohlzylinder
- 44: Einströmöffnung
- 46: Vorspannfeder
- 48: Führungshülse
- 50: Diffusor

## Patentansprüche

1. Behandlungssystem (1) zur Reinigung eines mit Biofilm verunreinigten Bauteils, insbesondere zur Reinigung von bakteriell verunreinigten Oberflächen von Knochenimplantaten oder Dentalimplantaten (20), mit einem in elektrischen Kontakt mit dem behandlungsbedürftigen Bauteil bringbaren, an einen ersten Pol einer elektrischen Versorgungseinheit (16) angeschlossenen Leiterelement (10), **gekennzeichnet durch** eine zur Ausbringung einer Behandlungsflüssigkeit vorgesehenen Medienkanüle (2), deren Innenraum elektrisch leitend mit dem zweiten Pol der elektrischen Versorgungseinheit (16) verbunden ist.

2. Behandlungssystem (1) nach Anspruch 1, bei dem das Leiterelement (10) und die Medienkanüle (2) in einem gemeinsamen Gehäuse (30) angeordnet sind.

3. Behandlungssystem (1) nach Anspruch 1 oder 2, bei dem die Auslassöffnung (4) der Medienkanüle (2) mit einem Verschluss (40) versehen ist, der selbsttätig dann öffnet, wenn die Auslassöffnung (4) mit einer Kraft von mehr als einer vorgebbaren Mindestkraft gegen eine Fläche angedrückt wird.

## Claims

1. A treatment system (1) for cleaning a component contaminated with a biofilm, in particular for cleaning bacterially contaminated surfaces of bone implants or dental implants (20), having a conductor element (10) that can be brought into electrical contact with the component needing treatment and is connected to a first pole of an electrical supply unit (16), **characterized by** a media cannula (2) provided for yielding a treatment fluid, whose inner space is connected in an electrically conductive manner with the second pole of the electrical supply unit (16).

2. The treatment system (1) of claim 1, wherein the conductor element (10) and the media cannula (2) are arranged in a common housing (30).

3. The treatment system (1) of claim 1 or 2, wherein the outlet opening (4) of the media cannula (2) is provided with a closure (40) which will automatically open when the outlet opening (4) is pressed against a surface with a force of more than a predefinable minimum force.

## Revendications

1. Système de traitement (1) conçu pour nettoyer un composant souillé par un biofilm, en particulier pour nettoyer des surfaces souillées bactériellement, d'implants osseux ou d'implants dentaire (20), ayant un élément conducteur (10) qui peut être mis en contact électrique avec le composant nécessitant le traitement et qui est relié à un premier pôle d'une unité d'alimentation électrique (16), **caractérisé par** une canule à milieu (2) pourvue pour délivrer un fluide de traitement, dont l'espace intérieur est relié de manière électriquement conductive au second pôle de l'unité d'alimentation électrique of the electrical supply unit (16).

2. Système de traitement (1) selon la revendication 1, dans lequel l'élément conducteur (10) et la canule à milieu (2) sont disposés dans un boîtier (30) commun.

3. Système de traitement (1) selon la revendication 1 ou 2, dans lequel l'orifice de sortie (4) de la canule à milieu (2) est pourvu d'une fermeture (40) qui ouvrira automatiquement lorsque l'orifice de sortie (4) est pressé contre une surface avec une force supérieure à une force minimum prédéfinie.
